# EUROPÄISCHE PATENTSCHRIFT

(11) **EP 1 013 176 B1**
(45) Veröffentlichungstag und Bekanntmachung des Hinweises auf die Patenterteilung: **04.08.2004**
(21) Anmeldenummer: 99123662.1
(22) Anmeldetag: 29.11.1999
(51) Int. Cl.: A23L 1/22, A61K 9/20

(54) **Zucker- und/oder zuckeralkoholhaltiges Matrixmaterial und Verfahren zu seiner Herstellung**
Sugar and/or sugar alcohol containing matrix material and method for preparing the same
Matériau pour matrice comprenant des sucres et/ou des alcools de sucre et procédé pour le préparer

(30) Priorität: 22.12.1998 AT 213098
(43) Veröffentlichungstag der Anmeldung: 28.06.2000
(73) Patentinhaber: Dr. Gergely & Co., 1053 Wien (AT)
(72) Erfinder: Gergely, Gerhard, Dr., 1053 Wien (AT); Gergely, Thomas, Dr., 1053 Wien (AT); Gergely, Irmgard, 1053 Wien (AT)
(74) Vertreter: Büchel, Kurt F.

(56) Entgegenhaltungen:
- EP-A- 0 076 115
- EP-A- 0 435 450
- EP-A- 0 629 393
- AT-B- 359 978
- DE-A- 3 635 864
- US-A- 4 031 255
- US-A- 5 370 881

## Beschreibung

Die Erfindung betrifft ein Matrixmaterial nach dem Oberbegriff des Anspruchs 1 und ein Verfahren zu seiner Herstellung. Ein Verfahren zur Herstellung einer festen Dispersion von festen Stoffen, wie pulverförmige Arzneistoffe oder Geschmacksträger, oder von flüssigen Stoffen, wie Aromen, Arzneistoffen sowie auch flüssigen Konzentraten oder Extrakten in Zucker ist beispielsweise in der AT-PS-359978 beschrieben. Dabei werden die einzukapselnden Stoffe unter Inertgasatmosphäre einem Gemisch mehrerer Zucker zugefügt, wodurch der zu schützende Stoff im Zuckermedium dispergiert und anschliessend mit einem gekühlten Medium in Berührung gebracht wird.

Nun hat sich aber im Laufe der praktischen Anwendung gezeigt, dass es je nach dem Verhältnis von z.B. Mannit, Sorbit und Glucose, bzw. der in der genannten AT-PS angeführten Zucker, wie Erythrit, Mannit, Sorbit, Fructose oder Glucose, entweder doch noch zu einer Kristallisation der Zuckermatrix kommt oder bei einem erhöhten Einsatz von Fructose oder Glucose das Produkt für die weitere Verarbeitung - speziell für die erforderliche Vermahlung - zu elastisch wird, sodass die Masse in der Mühle verklebt. Darüber hinaus kam es auch beim Einsatz von Fructose oder Glucose zu Verfärbungen, speziell bei der Verwendung in Kombination mit Alkalien, wie z.B. bei dem Einsatz in Kombination mit einer Brausebasis, bei der die Matrixbestandteile in Kontakt mit den alkalischen Brausebestandteilen kommen, wie z.B. mit Natriumbicarbonat oder -carbonat.

Die Aufgabenstellung der Erfindung war daher einerseits, die oben erwähnten Nachteile zu verhindern und eine verbesserte Zusammensetzung für eine Suspension von flüssigen Bestandteilen, wie z.B. Aromen, zu finden, bzw. auch feste Bestandteile besser zu verkapseln und das Verfahren zu optimieren. Dies gelang erfindungsgemäss erstmals überraschend durch die im Kennzeichen des Anspruchs 1 angeführten Merkmale. Bevorzugt ist der Einsatz von Gluconsäure-delta-Lacton. Vorteilhafte Weiterbildungen der Erfindung sind in den Kennzeichen der abhängigen Ansprüche beschrieben.

Zusätzlich wurde angestrebt, die empfindlichen Stoffe einerseits besser vor Oxidation andererseits vor Verseifung zu schützen, was speziell für Aromastoffe von Bedeutung ist. Aber nicht nur zur Verhinderung einer möglichen Kristallisation, sondern auch um den Einfluss der Oxidation sowie die Gegebenheiten für eine Verseifung von empfindlichen Wirkstoffen oder flüssigen Geschmacksträgern zu verhindern, hat sich erstaunlicherweise der Einsatz von Gluconsäure-delta-Lacton oder dergleichen Estern mit den Zuckeralkoholen in gemeinsamer Schmelze bewährt. Einerseits haben diese die Eigenschaft, die Kristallisation von Mannit bzw. von Mannit-Sorbit-Gemischen besonders gut zu verhindern; andererseits spalten sie bei leicht erhöhter Temperatur geringfügige Mengen an Gluconsäure ab, wodurch das Produkt im schwach sauren pH-Bereich gehalten wird. Dies wirkt daher einer Verseifung der empfindlichen Stoffe im Kontakt mit Alkalien entgegen und begünstigt so die Stabilität. Dem Matrixmaterial kann zusätzlich ein Oxidationsschutzmittel, wie z.B. Tocopherolacetat (Vitamin E-acetat), zugefügt werden, um für die gegen Luft- Sauerstoff empfindlichen Wirk- oder Aromastoffe einen zusätzlichen Schutz in die Matrix zu inkorporieren. Dafür sind relativ geringe Mengen ausreichend, etwa 0,01 - 0,005%.

Im Gegensatz zu der Verwendung von Glucose oder Fructose in einer Schmelze mit Mannit und Sorbit wird die eründungsgemässe Schmelze nicht so elastisch und lässt sich anschliessend gut zerkleinern, ohne dass es zu einem Verkleben der Mühle kommt. Dies wirkt sich auch positiv für das Herstellverfahren aus, sodass die Schmelze beim Austragen nicht mehr mit einem so stark gekühlten Medium in Berührung gebracht werden muss, um zu erstarren. Es ist daher nicht mehr erforderlich, dass die Temperatur der gekühlten Metallfläche höchstens 10, vorzugsweise 0°C beträgt, sondern es kann mit einer leichten Wasserkühlung bei 12 - 18°C eine ausreichende Erstarrung der Schmelze in der entsprechenden Zeit gewährleistet werden, sodass die in der Schmelze suspendierten Tröpfchen gleichmässig in der Schmelze erstarren und es nicht zu einer Abtrennung kommt. In die Schmelze kann auch noch Aerosil® zur besseren Verarbeitbarkeit beim anschliessenden Mahlen eingearbeitet werden, in einer Menge von 0,2 - 1 %.

Gluconsäure-delta-Lacton in einer Schmelze mit Sorbit und Mannit kann relativ hoch dosiert werden, in einer Menge von 15 oder sogar bis zu 35 Gew. % auf die fertige Schmelze bezogen. Besonders bewährt hat sich für die angestrebten Eigenschaften ein Gemisch aus Mannit, Sorbit und Gluconsäure-delta-Lacton, wobei auf 100 Gew.teile Schmelze eine relativ geringe Menge an Sorbit zum Einsatz kommen kann, etwa 1 bis 10%, Mannit hingegen kann in einer Menge von 50 - 80% eingesetzt werden.

Bei Gluconsäure-delta-Lacton muss während der Verarbeitung jedoch darauf Rücksicht genommen werden, dass es sich bei höheren Temperaturen schon während des Aufschmelzens in Gluconsäure zersetzen kann. Um dies zu verhindern, wird so vorgegangen, dass zunächst Mannit und Sorbit bei 190°C aufgeschmolzen werden und erst beim Abkühlen Gluconsäure-delta-Lacton hinzugefügt wird, wobei die zum Schmelzen des Gluconsäure-delta-Lactons notwendige Schmelzwärme dem geschmolzenen Mannit-Sorbit-Gemisch entzogen wird. Man gelangt dabei zu einer genau vorher bestimmten Abkühlung auf 150°C.

Wie bereits oben erwähnt, kann man - um spätere Verklebungen in der Mühle zu verhindern - der Schmelze vor dem Ausgiessen noch eine Menge von 0,2 - 0,8 Gew.teilen Aerosil® hinzufügen.

Diese Masse wird bei 135° C bereits stark viskos, sodass die Flüssigstoffe vermittels hoher Scherkräfte in einem Temperaturbereich von vorzugsweise 150 und 146°C eingebracht werden. Durch eine spezielle Schaltung wird das Gemisch bei dieser Temperatur gehalten und sodann auf ein umlaufendes Förderband aus teflonisiertem Gewebe mit einer Gesamtlänge von etwa 20 - 40 m aufgebracht. Der obere Bandteil läuft auf einer wassergekühlten Metallfläche von 12 - 15°C. Nach 10 - 20 m ist das Produkt so weit erstarrt, dass man es anschliessend durch eine Schlagvorrichtung zerkleinern und einer nachfolgenden Vermahlung zuführen kann.

Die so hergestellten Trockenaromen haben den Vorteil, durch viele Jahre hindurch stabil zu bleiben, da die im Inneren der Teilchen eingeschlossenen Öltröpfchen völlig luftabgeschlossen und daher von Oxidation oder anderen äusseren Einwirkungen bewahrt sind. Darüber hinaus bilden sich, wie ebenfalls bereits erwähnt, durch das schonende Aufschmelzen von Gluconsäure-delta-Lacton geringe Mengen an Gluconsäure; dadurch liegt in der festen, nicht kristallinen Schmelze auch eine optimale pH-Struktur vor, sodass die Matrix leicht sauer gehalten wird. Dadurch ist ein zusätzlicher Schutz gegen Verseifung gegeben.

Nach diesem Verfahren erübrigt sich auch eine Begasung durch Inertgas, da der Vorgang äusserst schnell und zügig abläuft.

Nachstehend einige Beispiele:

### Beispiel 1 (Verwendung der Schmelze für flüssige Aromen)

In einem Schmelzkessel werden 68 Gew.teile Mannit und 2 Gew.teile Sorbit aufgeschmolzen. Bei 190°C werden 30 Gew.teile Gluconsäure-delta-Lacton eingebracht und unter Dauerrühren aufgeschmolzen, wobei sich infolge der für das Aufschmelzen des Gluconsäure-delta-Lactons benötigten Schmelzwärme eine Temperatur von 148 - 150°C einstellt. Bei 150°C werden sodann 7 Gew.teile flüssiger Aromastoff unter Druck eingerührt; anschliessend wird die Schmelze auf ein gekühltes Band von ca. 12 - 15°C ausgegossen, wo sie innerhalb von 20 Minuten erstarrt, sodass es zu keiner Absonderung der Öle in der Schmelze kommen kann. Nach einiger Zeit kann die Schmelze grob gebrochen und anschliessend vermahlen werden.

### ( Beispiel 2 (Einbringen von festen Stoffen in die Schmelze)

In gleicher Weise kann das Einbringen eines festen Stoffes, wie z.B. eines temperaturstabilen Wirkstoffes, in die Schmelze erfolgen, wobei nach dem Aufschmelzen von Gluconsäure-delta-Lacton - z.B. bis zu 20 Gew.teile - fein mikronisierter Wirkstoff mit einem Hochdruckrührer in die Schmelze eingetragen und dispergiert werden. Die Schmelze wird anschliessend ausgegossen und auf einem Band zur Abkühlung gebracht.

### Beispiel 3:

Als Basis für die Verkapselung kann die Schmelze auch folgendermassen zusammengesetzt sein: 10 Gew.teile Sorbit werden mit 70 Gew.teilen Mannit bei 190°C aufgeschmolzen; es werden dann wie oben beschrieben 20 Gew.teile Gluconsäure-delta-Lacton zugefügt. In die resultierende Schmelze kann man flüssige und/oder feste Bestandteile einbringen, mit einem Turborührer verteilen, sowie anschliessend die Schmelze ausgiessen und zum Erstarren bringen. Die Schmelze kann anschliessend auf die gewünschte Korngrösse vermahien und gesiebt werden.

## Patentansprüche

1. Zucker- und/oder zuckeralkoholhältiges Matrixmaterial zur Einkapselung von festen oder flüssigen Stoffen, insbesondere von pharmazeutischen Wirkstoffen und/oder wenigstens einem pharmazeutisch zulässigen Aroma, wobei das Matrixmaterial eine Substanz enthält, die beim Abkühlen aus ihrer Schmelze deren Kristallisation weitgehend unterdrückt und - auf die Gesamtmenge des Matrixmaterials bezogen - in einer Menge von 10 bis 50, vorzugsweise von 20 bis 35 Gew.% vorliegt, **dadurch gekennzeichnet, dass** als kristallisationsunterdrückende Substanz der innere Ester einer Hydroxysäure, insbesondere Gluconsäure-delta-Lacton, vorliegt.

2. Matrixmaterial nach Anspruch 1, **dadurch gekennzeichnet, dass** das Matrixmaterial 0,1 bis 10, vorzugsweise 1 bis 3 Gew.% einer auch die Verseifung des Wirkstoffes bzw. des Aromas unterdrückenden Substanz, insbesondere einer pharmazeutisch zulässigen - vorzugsweise schwachen - Säure, wie z.B. Milchsäure oder Äpfelsäure, enthält.

3. Matrixmaterial nach einem der vorhergehenden Ansprüche, **dadurch gekennzeichnet, dass** das Matrixmaterial zusätzlich ein pharmazeutisch zulässiges Oxidationsschutzmittel, insbesondere Vitamin E, enthält.

4. Verfahren zur Herstellung eines Matrixmaterials nach einem der vorhergehenden Ansprüche, **dadurch gekennzeichnet, dass** man - gegebenenfalls die Mischung der - Zucker bzw. Zuckeralkohole auf eine Temperatur erhitzt, die über deren Schmelztemperatur und über der Schmelztemperatur der die Kristallisation bzw. die Verseifung unterdrückenden Substanz liegt, anschliessend unter Mischen die die Kristallisation bzw. die Verseifung unterdrückenden Substanz, und schliesslich - ebenfalls unter Mischen - den festen und/oder flüssigen Stoff, insbesondere den pharmazeutischen Wirkstoff und/oder wenigstens ein Aroma einbringt, worauf man das Gemisch abkühlt und gegebenenfalls zerkleinert bzw. vermahlt.

## Claims

1. Sugar and/or sugar alcohol containing matrix material for encapsulating solids and liquids, and more particularly pharmaceutically active substances and/or at least a pharmaceutically acceptable flavor, the matrix material containing a substance which upon cooling from the melt of the matrix material largely suppresses its crystallization and which relative to the total amount of matrix material is present in an amount of 10 to 50, preferably 20 to 35 wt.%, **characterized in that** the inner ester of a hydroxy acid, and more particularly gluconic acid delta-lactone, is present as the substance suppressing crystallization.

2. Matrix material according to claim 1, **characterized in that** the matrix material contains 0.1 to 10, preferably 1 to 3 wt.% of a substance also suppressing saponification of the active substance or flavor, more particularly that of a pharmaceutically acceptable acid, preferably weak, such as lactic or malic acid.

3. Matrix material according to one of the preceding claims, **characterized in that** the matrix material in addition contains a pharmaceutically acceptable antioxidant, more particularly vitamin E.

4. Method for preparing a matrix material according to one of the preceding claims, **characterized in that** the sugars or sugar alcohols, or where applicable their mixture, are heated to a temperature above their melting point and above the melting point of the substance suppressing crystallization or saponification, followed by adding while stirring, the substance suppressing the crystallization or saponification, and finally by adding the solid and/or liquid, and more particularly the pharmaceutically active substance and/or at least one flavor, again while stirring, then cooling the mixture and, where applicable, comminuting or grinding it.

## Revendications

1. Matériau pour matrice contenant des sucres et / ou des alcools de sucres, pour encapsuler des substances solides ou liquides, en particulier des substances pharmaceutiquement actives et / ou au moins un arôme pharmaceutiquement acceptable, où le matériau pour matrice contient une substance qui, lors du refroidissement de la masse fondue du matériau pour matrice, inhibe dans une large mesure la cristallisation de celle-ci et qui est présente en une quantité allant de 10 à 50 et, de préférence, de 20 à 35 % en poids de la quantité totale du matériau pour matrice, **caractérisé en ce que** la substance inhibant la cristallisation est l'ester interne d'un acide hydroxylique et, en particulier, la lactone du type delta de l'acide gluconique.

2. Matériau pour matrice selon la revendication 1, **caractérisé en ce que** le matériau pour matrice contient de 0,1 à 10 % et, de préférence, de 1 à 3 % en poids d'une substance qui est capable d'inhiber la saponification de la substance active ou de l'arôme et qui est, en particulier, un acide pharmaceutiquement acceptable et, de préférence, un acide faible, comme par exemple l'acide lactique ou l'acide malique.

3. Matériau pour matrice selon l'une des revendications précédentes, **caractérisée en ce que** le matériau pour matrice contient en plus, une substance pharmaceutiquement acceptable assurant une protection contre l'oxydation, en particulier la vitamine E.

4. Procédé de préparation d'un matériau pour matrice selon l'une des revendications précédentes, **caractérisé en ce que** l'on chauffe les sucres, les alcools de sucres ou, le cas échéant, un mélange de ceux-ci, à une température qui est supérieure à leur température de fusion et à la température de fusion de la substance capable d'inhiber la cristallisation ou la saponification et qu'ensuite on introduit - tout en mélangeant - la substance capable d'inhiber la cristallisation ou la saponification et enfin - également en agitant - la substance solide et / ou liquide, en particulier la substance pharmaceutiquement active et / ou au moins un arôme, suite à quoi le mélange est refroidi et, le cas échéant, broyé ou pulvérisé.
